(19) Europäisches Patentamt
European Patent Office
Office européen des brevets

(11) Publication number: 0 231 151
A2

(12) EUROPEAN PATENT APPLICATION

(21) Application number: 87810040.3

(22) Date of filing: 23.01.87

(51) Int. Cl.$^4$: **A 61 K 31/47**
A 61 K 31/48, A 61 K 37/02

(30) Priority: 31.01.86 GB 8602372

(43) Date of publication of application:
05.08.87 Bulletin 87/32

(84) Designated Contracting States: ES GR LU

(71) Applicant: SANDOZ AG
Lichtstrasse 35
CH-4002 Basel (CH)

(72) Inventor: Larson, Douglas
Talholzstrasse 28
CH-4103 Bottmingen (CH)

(54) **Immunosuppressant agents.**

(57) Low molecular weight quinolines, e.g. ergolines, have been found to have immunosuppressant properties.

EP 0 231 151 A2

## Description

### IMMUNOSUPPRESSANT AGENTS

The present invention relates to immunosuppressant agents which are quinoline derivatives.

It has now been shown that low molecular weight quinoline derivatives exhibit immunosuppressant activity and may be administered on their own, or e.g. co-administered with an immunosuppressant agent, e.g. a cyclosporin, allowing the dosage of immunosuppressant agent to be reduced.

Hereinafter these quinoline derivatives are referred to as compounds of the invention. The present invention provides in one aspect the use of a compound of the invention in the preparation of an immunosuppressant pharmaceutical composition. These compounds may be used for example in free base form or in pharmaceutically acceptable acid addition salt form, e.g. the hydrochloride, maleate or mesylate.

The compounds of the invention may comprise a nucleus of e.g. up to fused 4 rings including the quinoline moiety. The nucleus may bear substituents, e.g. side-chains. The compounds are low molecular weight in the sense that each side-chain does not contain any fused rings.

Examples of low molecular weight quinoline derivatives are benzoquinolines, particularly of formula I

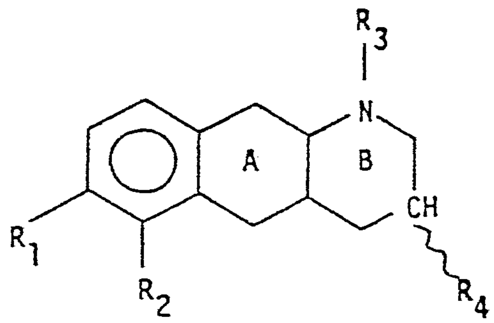

wherein the rings A and B are trans-fused and wherein $R_1$ and $R_2$ are each independently hydrogen, hydroxy or methoxy, with the proviso that $R_1$ and $R_2$ may not both be hydrogen.

$R_3$ is hydrogen or $C_{1-4}$alkyl;

$R_4$ is -COOH, $-CH_2OR_5$, $-CH_2CN$, $-CON(R_6)R_7$, $-CH_2SR_8$, $-NHSO_2N(R_9)R_{10}$ or $-NHCON(R_9)R_{10}$,

$R_5$ is hydrogen or $C_{1-3}$alkyl,

$R_7$ is hydrogen, $C_{1-3}$alkyl, phenyl or pyridyl, said phenyl or pyridyl being optionally substituted by halogen, methyl or methoxy or

$R_6$ and $R_7$ together are $-(CH_2)_4-$, $-(CH_2)_5-$ or $-(CH_2)_2-O-(CH_2)_2-$,

$R_8$ is $C_{1-4}$alkyl or pyridyl, said pyridyl being optionally substituted by halogen, methyl or methoxy, and

$R_9$ and $R_{10}$ area each independently hydrogen or $C_{1-3}$alkyl or together are $-(CH_2)_4-$ or $-(CH_2)_5-$,

as well as the physiologically-hydrolysable and -acceptable esters thereof.

These compounds are known from e.g. European Patent Publication 77754 (A2), the contents of which including all the examples are incorporated herein by reference.

The preferred compound thereof is N,N-diethyl-N'-[3α,4aα,10aβ)-1,2,3,4,4a,5,10,10a-octahydro-6-hydroxy-1-propyl-3-benzo[g]quino-linyl] sulfamide, also known as CV, (hereinafter compound A) preferably used as the hydrochloride.

Preferred compounds are low molecular weight ergot derivatives, i.e. compounds which do not have a peptide moiety in the 8 position, i.e. not ergopeptides. They may have for example an amino group, e.g. an acylamino, ureidio or sulphamino moiety or thiomethyl moiety in the 8 position which may be substituted by for example one or if desired two ($C_{1-4}$)alkyl groups. Conveniently these have a single bond in the 9,10 position of the ergoline nucleus.

The preferred compounds are 8α-sulphamoylamino ergolines. These may be based on the formula:-

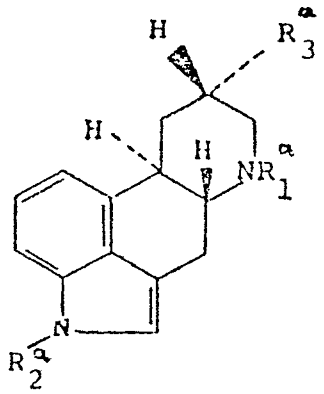

Ia

wherein $R_1{}^a$ inter alia is $(C_{1-4})$alkyl,
$R_2{}^a$ inter alia is H or $(C_{1-4})$alkyl,
$R_3{}^\alpha$ inter alia is $-NHSO_2N[(C_{1-4})alkyl]_2$

The preferred Examples include:-

a) 1,6-dimethyl-8α-(N,N-dimethylsulphamoylamino)-ergoline-I (also known as Mesulergine hereinafter compound B);

b) 6-n-propyl-8α-(N,N,-diethylsulphamoylamino)-ergoline-I (N,N-diethyl-N'-(6-propylergolin-8α-yl)sulfamide) preferably used as the hydrochloride, also known as CQP, (hereinafter compound C).

c) N,N-diethyl-N'-[(8α)-1-ethyl-6-methyl-ergolin-8-yl]-sulfamide preferably used as the hydrochloride, (hereinafter compound D).

The most preferred example is (b), i.e. compound C.

Other preferred compounds include:-

i) 3-(9,10-didehydro-6-methyl-ergolin-8α-yl)-1,1-diethyl-urea (also known as Lisuride preferably used as the hydrogen maleate);

ii) 6-n-propyl-8α-methylmercaptomethyl-ergoline-I (also known as Pergolide preferably used as the mesylate);

iii) Transhydrolisuride also known as terguride having the chemical name 3-(6-methyl-ergolin--8α-yl)-1,1-diethyl-urea, published e.g. in DOS 3135305 and 3124714.

iv) 6-n-propyldihydro-lisuride also known as proterguride having the chemical name 3-(6-n-propyl-ergolin-8α-yl)-1,1-diethyl-urea.

v) 6-and 2-substituted, e.g. 6-n-propyl and/or 2-methyl or bromo derivatives of terguride, lisuride and proterguride e.g. as published in European Patent Publication No. 21206 (A.1) and 160842 (A.1) the contents of which especially the examples and pharmacological data thereof, are incorporated herein by reference. Examples include 2-bromerguride, also known as 2- bromolisuride, preferably used in the form of the hydrochloride.

vi) Metergoline, also known as (+)-N-(carboxy)-1-methyl-9,10-dihydrolysergamine benzyl ester.

vii) dosergoside, also known as N-(1S,2R,3E)-2-hydroxy-1-(hydroxymethyl)-3-heptadecanyl)-6-methylergoline-8-beta-carboxamide.

viii) FCE-21336 also known as 1-ethyl-3-(3'-dimethylaminopropyl)-3-(6-alkyl-ergoline-8'-beta-carbonyl)-urea preferably used as the diphosphate.

ix) GYKI-32887 also known as 6-methyl-8-(N-mesyl-N-2-azidoethyl) ergolene preferably used as the bimaleate, e.g. as disclosed in USP 4,299,836.

Groups of compounds include:-

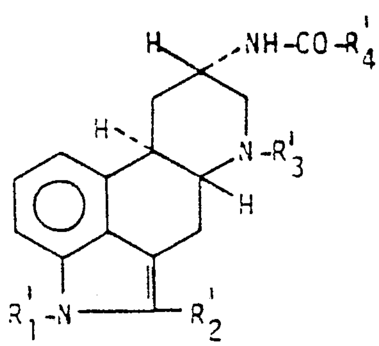

(I')

wherein
$R_1'$ is hydrogen or $C_{1-4}$alkyl,

$R_2'$ is hydrogen, chlorine, bromine or methyl,
$R_3'$ is $C_{1-5}$alkyl or $C_{3-5}$alkenyl in which the double bond is not at the carbon atom adjacent to the nitrogen atom, and
$R_4'$ is $C_{3-7}$alkyl; $C_{3-7}$cycloalkyl; adamantyl; phenyl; phenyl substituted by one or more members selected from the group consisting of $C_{1-3}$alkyl, $C_{1-3}$alkoxy, $C_{1-3}$alkylthio, trifluoromethyl, hydroxy, nitro, amino and mono- and di-($C_{1-3}$alkyl)-amino; or phenyl bearing a condensed non-aromatic, heterocyclic ring having 5- or 6-ring members including 1 or 2 hetero atoms selected from the group consisting of oxygen and sulphur, published in GB 2,152,507 A, the contents of which especially the examples and pharmacological data thereof are incorporated herein by reference, e.g.
(5R,8S,10R)-2,6-dimethyl-8α-pivaloylamino-ergoline (hereinafter compound E) preferably used as the hydrochloride, and the 2-chloro derivative, (5R,8S,10R)-2-chloro-6-methyl-8α-pivaloylamino-ergoline.

Other examples include a compound of formula I″

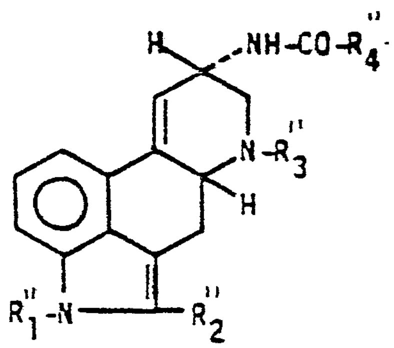

(II'')

wherein $R_1''$ is hydrogen or $C_{1-4}$alkyl,
$R_2''$ is hydrogen, chlorine,, bromine or methyl,
$R_3''$ is $C_{1-5}$alkyl or $C_{3-5}$alkenyl in which the double bond is not at the carbon atom adjacent to the nitrogen atom, and
$R_4$ is $C_{1-7}$alkyl; $C_{3-7}$cycloalkyl; adamantyl; phenyl; phenyl substituted by one or more members selected from the group consisting of $C_{1-3}$alkyl, $C_{1-3}$alkoxy, $C_{1-3}$alkylthio, trifluoromethy, hydroxy, nitro, amino and mono- and di-($C_{1-3}$alkyl)-amino; or phenyl fused with a non-aromatic, heterocyclic ring having 5- or 6-ring members including 1 or 2 hetero atoms selected from the group consisting of oxygen and/or sulphur,
with the proviso that when $R_2''$ is hydrogen, neither $R_3''$ nor $R_4''$ is methyl,
e.g. the compounds wherein $R_1'' = H$ $R_2'' = Br$ or especially $CH_3''$, $R_3'' = CH_3$ and $R_4''$ = tert butyl, filed off German Application P 3447383.1 filed 24 Dec 1984 as English application No. 8531420, now published as G.B.Application 2169291 A and also in other countries, the contents of which including all the examples thereof are incorporated herein by reference.

Many of the above mentioned compounds are published as prolactin secretion inhibitors, some of which are extremely potent and have long lasting activity. The potency of the compounds as prolactin secretion inhibitors can be determined in standard tests, e.g. by lowering basal serum prolactin levels in the male rat.

Results with representative compounds include:-

| Compound | $ID_{50}$ mg/kg after 4 hours |
|---|---|
| A | 0.03 |
| B | 0.003 |
| C | 0.001 |

A group of compounds comprises compounds which are $D_2$ agonists without any antagonistic activity. Another group of compounds includes those compounds which are serotonin agonists, e.g. as indicated in standard in vitro binding tests and by activation of 5-HT sensitive adenylate cyclase of the liver fluke according to the method of R.Markstein, Neurochemical effects of some ergot derivatives: A basis for their antiparkinson actions. J.Neural transm 51:39, (1981).

Examples of such compounds include compound C and pergolide. Compound C, pergolide and 5-HT have efficacies of 90,80 and 100 respectively and $pD_2$ [log $EC_{50}$] of 6.9, 6.9 and 7.7 respectively in this test on adenylate cyclase stimulated by serotonin.

As indicated below surprisingly the potency of a compound of the invention as a prolactin secretion inhibitor may serve as a guide to as to the potency of its immunosuppressant effect, but, at least before the date of this

invention, there is no evidence of a direct correlation between their ability to lower prolactin levels and their immunosuppressant effect.

Indeed the potency of these compounds as prolactin secretion inhibitors and their long duration of action with this property may well have masked the observation of their interesting properties in other directions, especially on the basis of in vitro testing. We have effected exhaustive testing with these compounds of the invention and have found that they possess particularly interesting immunosuppressant properties often at lower than expected doses.

The compoundse of the invention inhibit immuno-response activity as indicated in standard ex-vivo and in-vivo tests.

TEST 1: ANTI-BODY PRODUCING CELL DEVELOPMENT IN THE MOUSE (PFC TEST)

The compounds of the invention reduce the anti-body titer to SRBC (Sheep red blood cells) in plaque forming cell (PFC) assays. These methods permit the quantitation of the number of splenic lymphocytes forming lytic antibodies to the SRBC and the distinction between IgM and IgG producing cells.

The intravenous injection of SRBC (5 x $10^7$) in 0.2 ml i.v. served as the primary immunization antigen source in the mouse studies. The mice in these studies were female C57 black of ca 20 grams body weight. All animals were pretreated with the respective compounds for one day prior to the SRBC treatment and daily treated thereafter at 11.00 h for a total of 6 days. The spleens were removed for the determination of PFC numbers to the SRBC antigen.

The spleen cells were suspended in Hepes-buffered Balanced Salt Solution (HBSS), washed once, and resuspended in a volume adjusted according to the expected number of plaque forming cells. The direct quantitiation of IgM-PFC was performed by plaquing 0.1 ml of the diluted splenic cells with 0.025 ml of SRBC (30%), 0.3 ml Agar (0.5% in HBSS), and 0.025 ml complement (guinea pig serum) and plated on Petri dishes. An indirect method of IgG-PFC quantitation was performed with a monoclonal antibody to the IgM (1:4 dilution) plus an anti-gama polyspecific antiserum (1:800) to permit complement fixation. The splenic lymphocytes from each mouse spleen were plated to quantitate IgM-and IgG-PFC in duplicate. All plates were incubated for 4 hours at 37° C and then stored overnight in humidified chambers at 4° C. Plaques were counted by hand with the use of a plaque counter.

Representative results are as follows:-

PLAQUE FORMING ASSAY EFFECT

TEST A

| TREATMENT | IgM (PFC x$10^3$ per spleen) | % of control | IgG (PFC x $10^3$ per spleen) | % of control |
|---|---|---|---|---|
| CONTROL | 195.6 ±33.3 | --- | 46.2 ±11.6 | --- |
| Cyclosporine* 36 mg/kg/ day s.c. | 98.6* ±18.2 | -49.6 | 24.4* ±10.6 | -47.2 |
| Compound A 1.0 mg/kg/ day s.c. | 87.0* ±26.5 | -55.4 | 13.6* ±10.3 | -71.6 |

* also known as ciclosporin and cyclosporine A

TEST B

| TREATMENT | IgM (PFC x $10^3$ per spleen) | % of control |
|---|---|---|
| CONTROL | 329.6 | |
| Cyclosporin 60 mg/kg/day p.o. | 180.9 | -45% |
| Compound C 1 mg/kg/day p.o. | 327.0 | -1% |
| Compound C 0.1 mg/kg/day p.o. | 244.4 | -26% |
| Compound C 0.01 mg/kg/day p.o. | 167.2 | -49% |

In this test compound A based at a dosage of 1 mg/kg/day over 6 days administered s.c. had an effect of the same order as Cyclosporine at 36 mg/kg/day under similar conditions. Compound C at a dosage of 0.01 mg/kg/day p.o. over 6 days administered p.o. had an effect which was of the same order as Cyclosporine at 60 mg/kg/day under similar conditions. The immunosuppressant effect of compound C decreased at higher dosages. The dosage of other compounds of the invention may be ascertained by routine experimentation bearing in mind the relevant potencies of prolactin secretion inhibition, and that an immunosuppressant activity may not be observed at low and high dosages. Examples of dosages lie in the range 0.01 to 10 mg/kg e.g. up to 1 mg/kg, p.o. and s.c.

TEST 2: MIXED LYMPHOCYTE REACTION TEST IN MICE

The ability of the compounds of the invention to reduce lymphocyte responsiveness to allogenic stimulation is measured in the mixed lymphocyte reaction test (MLR) wherein irradiated allogenic cells from stimulator mice induce a proliferative response in responder histo-incompatible cells.

1ST VARIANTE:

Responder mice (Balb/c female, 8-10 weeks old) are treated sub-cutaneously for five days with the compounds of the invention. Equal numbers of murine stimulator lymphocytes (from stimulator, CBA, mice; lymphocytes irradiated in vitro with 200 R) and effector lymphocytes from the treated animals or control animals are incubated for five days. The 3H thymidine incorporated into lymphocyte DNA was used as a parameter for cell proliferation in response to an allogenic stimulus. [See S.S.Rich et al, J.Exp.Medicine, (1974), 140, 1588-1693].

Representative results obtained are:-

| | dpm x $10^3$/10 cells ± SD |
|---|---|
| Control | 131192 ± 12344 |
| Compound E at 5 mg/kg s.c. | 103218 ± 21497 |

Thus compound E inhibited the response by about 21%. The dosages for other compounds are found by experimentation, bearing in mind the relative potencies in inhibiting prolactin secretion, and that an immunosuppressant activity may not be observed at low and at high dosages. Examples of ranges are e.g. 1 to 10 mg/kg s.c.

2ND VARIANTE:

The compounds furthermore inhibit the immuno-responsiveness of effector lymphocytes to histoincompatible (allogenic) lymphocytes in a variante of the above two way MLR (mixed lymphocyte reaction) test.

Mice of two histo-incompatible strains (Balb/c and CBA) (6-8 weeks old) were once daily treated s.c. with a compound of the invention for at least 7 days. The spleens are removed 3 hours after the last drug administration. The MLR test is effected with 0.5 x $10^6$ spleen cells from Balb/c mice and 0.5 x $10^6$ spleen cells from CBA mice the latter having been irradiated with 2000 rad. The spleen cells of the Balb-c mice are incubated with the allogenic CBA-spleen cells for 4 days. Radioactive thymidine is added and after 16 hours the incorporation of $^3$H-thymidine in the DNA of the mouse spleen cells was measured.

Result obtained with Compound C

| Dose | Change from control |
|---|---|
| 5 mg/kg | -20% |
| 0.5 mg/kg | -50% |
| 0.05 mg/kg | -71% |
| (Cyclosporin 100 mg/kg) | -53% |

Dosages of the other compounds are found by experimentation bearing in mind relative potencies in inhibiting prolactin secretion, and that an immunosuppressant activity may not be observed at low and at high dosages. Examples of ranges are e.g. 0.01 to 10 mg/kg, e.g. 0.01 to 1 mg/kg.

TEST 3: FREUND'S ADJUVANT ARTHRITIS IN THE RAT

One test model is a rodent model of developing autoimmune adjuvant arthritis. An inhibition of the development of adjuvant arthritis is observed.

In one variante, Myobacterium Butyricum purchased from Difco Laboratories, Detroit, Michigan, was prepared in an emulsion with paraffin subliquidium PHHVI (Siegried Zofingen, Switzerland) at a concentration of 6 mg/ml. During ether anaesthesia a volume of 0.1 ml of this emulsion was injected with a 21 g 1-1/2 inch needle intracutaneously at the base of the tail of male Sprague-Dawley rats (ca 150-175 g). Arthritis occurred in 95% to 100% of the hind legs in the positive control animals. The hind limb (mid-tibial region) thickness was measured with a linear guage (Mitutoyo) coupled to an electronic digital readout (Mitutoyo).

100% of a control group developed adjuvant arthritis. The compounds of the invention are administered by means of a sub-cutaneously sustained osmotic pump, over 7 days or 14 days to groups of 5 animals. Progression of the arthritis and the inhibiting effect of a representative compound, compound B, at 0.1 mg/kg/day are as follows:-

Hind Limb Thickness (mm)

| Day | Control Group | Compound B 7 days dosing | Compound B 14 days dosing |
|---|---|---|---|
| 0 | 6.1 | 6.1 | 6.0 |
| 3 | 6.2 | 6.2 | 6.2 |
| 6 | 6.3 | 6.2 | 6.2 |
| 9 | 7.0 | 6.5 | 6.7 |
| 12 | 7.7 | 6.9 | 7.7 |
| 15 | 7.8 | 7.6 | 7.8 |
| 18 | 8.9 | 7.9 | 7.9 |
| 21 | 9.5 | 8.2 | 8.2 |

The dose of other compounds may be ascertained by routine experimentation bearing in mind the relative potencies of prolactin secretion inhibiting activity. Examples of doses are from 0.1 to 1 mg/kg/day s.c.

TEST 4: LOCAL GRAFT VERSUS HOST (GvH) IN THE RAT

Spleen cells (ca 1 x $10^7$) are taken from 6 week old Wistar/Furth/(W/F) rats and are injected into the left hind paw of female (F 344 x WF) F1 rats weighing about 100 g. The rats had been treated daily with the compound from 7 days before the injection and are treated daily for 3 days thereafter. The difference in weight between the left and right lymph nodes is taken as the parameter for evaluating the reaction.

Results obtained with representative compounds are:-

| Weight of lymph node Control | Weight lymph node | Percentage Difference |
|---|---|---|
| | Compound A 0.01 mg/kg/day | |
| ca 63 mg | ca 50 mg | -21% |
| | Compound C 0.001 mg/kg/day | |
| ca 33 mg | 24 mg | -28% |
| | Compound C 0.01 mg/kg/day | |
| ca 33 mg | 19 mg | -42% |

| | | |
|---|---|---|
| | Compound C<br>0.1 mg/kg/day | |
| ca 33 mg | 18 mg | -45% |
| | Compound E<br>5 mg/kg/day | |
| ca 55 mg | ca 39 mg | -41% |

As can be observed compound C is active in a dose dependent manner.

The dose of other compounds may be ascertained by routine experimentation bearing in mind the relative potencies of prolactin secretion inhibiting activity, and that an immunosuppressant activity may not be observed at low and at high dosages. Examples of doses are from about 0.001 to 5 mg/kg7day.

TEST 5: BIOSYNTHETIC RATE IN THE RAT

The compounds of the invention inhibit the biosynthetic rate of an appropriate marker for hormones, mitogens and compounds namely ornithine decarboxylase (ODC) in the thymus, spleen, bone marrow and anterior pituitary e.g. in the immune stimulated rat.

The marker used in this study to determine the biosynthetic rate of the lymphoid and endocrine tissues was ornithine decarboxylase (ODC). ODC, the rate-limiting enzyme in polyamine biosynthesis, serves as a early marker of $G_1$ cell cycle progression of proliferating tissues and increased biosynthesis in secretory tissues, in response to trophic or stimulatory substances. There is a peak activity of ODC subsequent to stimulation in vivo within 4 hours - making this enzyme a useful intracellular marker for understanding the relationship between agonists and target tissue response. The ODC activity of lymphoid tissues appears to relate to a functional response to the immune system.

In a typical test method KFM male rats (150 - 175 g) were treated with the compound of the invention one day prior to the administration of Complete Freund's adjuvant in analogous manner to that described above. Administration of the compound was repeated once time per day afterwards. Two days later the animals were decapitated, the tissues removed and frozen in liquid nitrogen. The ODC activities (pmol/30 min./mg protein) are measured. The ODC activity was determined by measuring the liberation of $14CO_2$ from L-(1-14C)-ornithine as described by Snyder et al Proc.Natl.Acad.Sci.60:1420, 1968.

Results obtained with Compound C are as follows:-

| | Control | Freund's Treatment | Compound C 0.01 mg/kg | 0.1 mg/kg | 0.5 mg/kg |
|---|---|---|---|---|---|
| Thymus | 291.8 | 253.8 | 98.80 | 171.40 | 177.0 |
| SEM | 32.95 | 24.33 | 24.15 | 19.48 | 53.70 |
| Spleen | 24.83 | 34.88 | 12.14 | 34.62 | 37.96 |
| SEM | 3.77 | 6.54 | 1.83 | 5.35 | 11.89 |
| Lymph Nodes | 40.70 | 64.40 | 43.10 | 107.30 | 103.90 |
| Bone Marrow | 37.20 | 27.10 | 17.40 | 45.80 | 43.40 |
| Adrenal Medulla | 15.50 | 7.40 | 17.10 | 180.0 | 95.0 |
| Anterior Pituitary | 170.40 | 151.30 | 32.60 | 33.70 | 41.20 |

As can be seen compound C, at doses at which it possesses potent immuno inhibitory properties, e.g. 0.01 mg/kg,inhibits the biosynthetic rates. At higher doses compound C stimulated ODC activity in the adrenal medulla, and the immunosuppressant effect was less.

Toxicity data for some of the comounds of the invention have been published. Such data may be determined by routine experimentation.

TOXICITY DATA for representative compounds

NTEL = No toxic effect level. Acute studies were over 7 or 14 days after i.v. or p.o. administration respectively unless otherwise mentioned.

Compound A: acute $LD_{50}$ (mouse) 17 mg/kg i.v. 357 mg/kg p.o.
NTEL (4 weeks) rats between 0.2 and 0.57 mg/kg/day p.o.
NTEL (4 weeks) dogs between 0.2 and 0.06 mg/kg/day p.o.

Compound B: Similar to compound E

Compound C: acute $LD_{50}$ (mouse) 45 mg/kg i.v. 69 mg/kg p.o.
NTEL (4 weeks) rats 0.19 mg/kg/day p.o.
NTEL (4 weeks) dogs 0.2 mg/kg/day p.o.

Compound D: acute $LD_{50}$ (mouse) 44 mg/kg i.v. 992 mg/kg p.o.
NTEL (26 weeks) rats between 5 and 17 mg/kg/day p.o.
NTEL (26 weeks) dogs between 0.3 mg/kg/day p.o.

Compound E: Well tolerated over 4 weeks administered to Beagle dogs up to 3 mg/kg/day p.o.

In view of their immunosuppressive activity, against both a primary and secondary immuno-response and against both IgM and IgG- PFC, the compounds of the invention are useful for the prophylaxis and treatment of disease and conditions requiring a reduction of the immune response, particularly mediated through the T and/or B-cells. Thus they may be used to suppress the proliferation of lymphocytes and immunocytes, e.g. in preventing the rejection of transplants, e.g. skin, bone-marrow and kidney transplants, treating and preventing graft versus host disease and especially in the treatment of auto-immune diseases.

The compounds are useful not only in vascular autoimmune diseases but also in a variety of autoimmune diseases relating to tissues and other organs and platelets.

Specific auto-immune diseases for which the compounds of the invention are useful include those which are spontaneous in nature, (e.g. not drug induced). Such autoimmune diseases may be characterised by specific antibodies. These include all of those for which treatment with Cyclosporine has been proposed or used, for example, aplastic anaemia, pure red cell anaemia, idiopathic thrombocytopenia (e.g. ideopathic thrombocytopenia purpura), systemic lupus erythematosus, polychondritis, scleroderma, Wegener granulamatosis, dermatomyositis, chronic active hepatitis, myasthenia gravis, psoriasis, Stevens-Johnson syndrome, sprue, e.g. idiopathic sprue, Crohn's disease, Graves Disease, Graves opthalmopathy, sarcoidosis, multiple sclerosis, primary billiary cirrhosis, primary juvenile diabetes (Diabetes type I), uveitis e.g. uveitis posterior and uveitis anterior, nephrotic syndrome, glomerular nephritis, interstitial lung fibrosis and psoriatic arthritis.

Particularly interesting indications include psoriasis and arthritis, multiple sclerosis, Diabetes type I, Grave's disease, uveitis and myasthemia gravis.

They are further useful for treatment of inflammatory conditions, e.g. arthritis and rheumatic disease with an autoimmune component and further the treatment of protozoal infections e.g. malaria, and schistomasis.

For the above-mentioned uses the dose will, of course, vary depending on the mode of administration, the compound employed, the particular condition to be treated and the therapy desired. In general, however, satisfactory results are obtained when administered at dosage sufficient to produce a long lasting and continuous prolactin secretion inhibition, e.g. to under 2 nanograms prolactin per ml blood plasma or at doses up to about 2 times higher in order to suppress any sudden prolactin surges. Indeed doses up to one tenth lower than the prolactin inhibiting doses are indicated to be effective in the immunosuppressant indication.

For the larger primates, in particular humans, an indicated daily dose is from about 0.001 to 10 mg (e.g. 0.1 to 10 mg) p.o. for the compounds of the invention. This may be conveniently administered for example in divided doses up to 4 times a day or preferably in sustained release form. Unit dosage forms contain for example from about 0.0002 to about 10 mg of the compounds.

The exact dosage to be administered may further depend on the relative potency to Cyclosporine A in the above tests. On this basis, for example it has been estimated that compound A would be administered at a dose of about 0.1 to 1 mg per day, e.g. 0.5 mg, p.o.

The preferred compound is compound C. This produces in humans a long-lasting prolactin secretion inhibiting effect at 0.01 mg to 0.03 mg p.o. per day. Indicated doses for an immuno suppressant effect are from 0.005 to 0.03 mg p.o.

The compounds of the invention may be administered in free base form or in pharmaceutically acceptable acid addition salt form. Such compositions are in general known and may be produced in conventional manner. The compounds may be administered by any conventional route in particular enterally preferably orally, e.g. in the form of capsules or tablets, or if appropriate parenterally in the form of injectable solutions or suspensions.

An example of a capsule composition containing compound A is as follows:-

| | | |
|---|---|---|
| Compound A (0.1 mg base) as hydrochloride | | 0.1092 mg |
| Corn starch | | 175.3908 mg |
| Lactose (200 mesh) | | 120.0 mg |
| Silica | | 1.5 mg |
| Magnesium stearate | | 3.0 mg |
| | Total | 300 mg |

An example of a capsule composition containing the preferred compound of the invention compound C is as follows:-

| | |
|---|---|
| Compound C in hydrochloride salt form | 0.02725* mg |
| Mannitol | 107.47275 mg |
| Corn Starch | 80.0 mg |
| Silica (Brand Aerosil 200) | 0.5 mg |
| | 188 mg |

(*corresponding to 0.025 mg free base)

Capsules containing lower amounts of the compound C, e.g. 0.005 mg may be made in analogous manner.

The present invention provides also a novel means of therapy, comprising conjugate administration of i) an immunosuppressant agent, e.g. a cyclosporin, in particular Cyclosporine and ii) a compound of the invention.

Immunosuppressant agents include azathioprine, cyclophosphamide, steroids e.g. methylprednisolone and Ciamexon.

The cyclosporins comprise a class of structurally distinct, cyclic, poly-methylated undecapeptides having valuable pharmacological, in particular immunosuppressive, antiinflammatory and anti-protozoal activity. The first of the cyclosporins to be isolated and the "parent" compound of the class, is the naturally occurring fungal metabolite Cyclosporine, also known as cyclosporin A, the production and properties of which are described e.g. in US Patent No, 4,117,118. Since the original discovery of Cyclosporine a wide variety of naturally occurring cyclosporins have been isolated and identified and many further non-natural cyclosporins have been prepared by synthetic or semi-synthetic means or by the application of modified culture techniques. The class comprised by the cyclosporins is thus now substantial and includes, for example, the naturally occurring cyclosporins (Thr$^2$). (Val$^2$)- and (Nva$^2$)-Cyclosporine (also known as cyclosprins C, D and G respectively) as well as various semi-synthetic derivatives thereof, such as their dihydro derivatives (e.g. as disclosed in US Patent Nos. 4,108,985; 4,210,581 and 4,220,641) including e.g. (Dihydro-MeBmt$^1$)-(Val$^2$)-Cyclosporine (also known as dihydrocyclosporin D) and other natural and artificial cyclosporins such as those disclosed in European Patnt Publication No. 0,058,134 81, for example [(D)-Ser$^8$]-[in accordance with now conventional nomenclature for the cyclosporins, these are defined herein by reference to the structure of Cyclosporine (i.e. cyclosporin A). This is done by first indicating those residues in the molecule which differ from those present in Cyclosporine and then applying the term "Cyclosporine" to characterise the remaining residues which are identical to those present in Cyclosporine. Cyclosporine has the formula I

| A- | B- | Sar- | MeLeu- | Val- | MeLeu- | Ala- | (D)Ala- | MeLeu- | MeLeu- | MeVal | |
|---|---|---|---|---|---|---|---|---|---|---|---|
| 1 | 2 | 3 | 3 | 5 | 6 | 7 | 8 | 9 | 10 | 11 | (I) |

wherein A represents the -MeBmt- [N-methyl-(4R)-4-but-2E-en-1-yl-4-methyl-(L)threonyl] residue of formula II

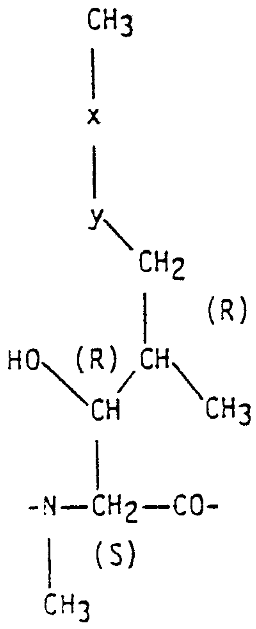

in which -x-y- is -CH=CH- (trans), and B is -α-Abu-. Accordingly ($Thr^2$)-Cyclosporine (cyclosporin C) is the compound of formula I, wherein A has the meaning given above and B is -Thr-, and (Dihydro-$MeBmt^1$)-($Val^2$)-Cyclosporine (dihydrocyclosporin D) is the compound of formula I, wherein A represent the -dihydro-MeBmt- residue of formula II above in which -x-y- is $-CH_2-CH_2-$and B is -Val-].

As the "parent" compound of the class, Cyclosporine has so far received the most attention. The primary area of clinical investigation for Cyclosporine has been as an immunosuppressive agent, in particular in relation to its application to recipients of organ transplants, e.g. heart, lung, combined heart-lung, liver, kidney, pancreatic, bone-marrow, skin and corneal transplants and, in particular, allogenic organ transplants. In this field Cyclosporine has achieved a remarkable success and reputation and is now commercially available and widely employed in clinic.

At the same time, applicability of Cyclosporine to various autoimmune diseases and to inflammatory conditions, in particular inflammatory conditions with an aetiology including an auto-immune component such as arthritis and rheumatic diseases, has been intensive and reports and results in vitro, in animal models and in clinical trials are wide-spread in the literature.

A further area of investigation has been potential applicability as an anti-parasitic, in particular anti-protozoal agents, with possible uses suggested including malaria treatment and treatment of schistosomiasis.

Other cyclosporins exhibit the same overall pharmacological utility as Cyclosporine and various proposals for application, in particular in one or other of the above identified indications, are prevelant in the literature.

Despite the very major contribution which Cyclosporine has made to the art, in particular in the field of transplant surgery and despite its wide acceptance and success in clinic, an obvious negative feature has been its reported organ toxicity, e.g. hepatoxicity and nephrotoxicity. In clinic nephrotoxicity has generally proved of more common concern and while it is evident that in those relatively few cases where a problem arises, nephrotoxicity is in any event dose-related, reversible and not progressive under long term treatment (clinically a problem is only likely to occur, if at all, in the early phase of Cyclosporine therapy, e.g. immediately subsequent to transplant when differentiation between rejection and nephrotoxicity is difficult) means of reducing this particular side effect and other related side effects of organ toxicity which might possibly occur would clearly be of major benefit. Thus apart from making Cyclosporine therapy more generally acceptable, it would reduce control requirements, e.g. immediately post-transplant, as well as the occasional necessity of employing mixed immunosuppressive therapy (e.g. drug-switching), for example in patients showing especially marked nephrotoxic reactions.

Similarly, while individual cyclosporins other than Cyclosporine may be found to exhibit considerably less toxic, e.g. nephrotoxic, side effects than Cyclosporine or indeed to be generally free of such side reactions, in so far as organ toxicity is or may be a consideration in relation to their clinical application, a means for meeting this problem would equally be of considerable benefit.

Similar considerations as discussed above to cyclosporins apply to other immunosuppressant agents. Administration leads to side effects, e.g. by toxicity problems, lymphoma, weight depression etc.

In accordance with the present invention, it has now, surprisingly, been found that immuno-suppressive effectiveness of immunosuppressant agents,e.g.cyclosporins, in particular Cyclosporine, is unexpectedly and advantageously enhanced by conjugate administration of compounds of the invention. More particularly, it has been found that conjugate administration of immuno-suppressant agents and compounds of the invention unexpectedly provides greater immunosuppressive potency that the sum potency of the individual

components (super-additive synergism).

Conjugate administration of compounds of the invention with immunosuppressant agents accordingly provides a means enabling significant reduction of immunosuppressant agent levels immunosuppressant agent otherwise required for immunosuppressive utility, e.g. in suppressing organ transplant rejection, in treating auto- immune disease as well as in treating inflammatory conditions with an aetology including an auto-immune component. By enabling dosage reduction of immunosuppressant agent, the present invention has the most important advantage of providing a means for obviating or reducing side effects, e.g. organ damage, consequent to administration of immunosuppressant agent during immunosuppressive therapy.

In accordance with the foregoing, the present invention provides a method:

1. for effecting immunosuppression, for example:

1.1 for preventing transplant rejection, e.g. in recipients of organ transplants, e.g. of any of the specific types hereinbefore set forth: or

1.2 for treating auto-immune disease, e.g. for treating any of the specific auto-immune disease hereinbefore set forth; or

2. for treating inflammatory conditions, in particular inflammatory conditions with an aetiology including an auto-immune component, for example, arthritis and rheumatic disease; or

3. for the treatment of protozoal infection, e.g. for the treatment of malaria,

in a subject in need of such treatment, which method comprises administering to said subject an effective amount of

a) immunosuppresant agents, e.g.a cyclosporin, e.g. cyclosporin, e.g. cyclosporin G or Cyclosporin, and

b) a compound of the invention.

Having regard to the synergistic interaction of components a) and b) with respect to immuno-suppression, the amounts of a) and b) required in accordance with the particular method defined under 1. above or, where a condition with an aetiology including an auto-immune component is concerned, in accordance with the particular method defined under 2. above, will conjointly comprise an immuno-suppressively effective amount. In addition, where residual organ toxicity or other side effects from the immunosuppressant agent is a consideration [e.g. despite lowered dosages of a) required consequential to administation of b)], the amount of b) required will suitably suffice to reduce such residual toxicity.

In relation to the particular methods defined under 2. and 3. above, the amount of a) required will comprise 2. an anti-inflammatorially effective and 3. an anti-protozoally effective amount respectively and the amount of b) required will be an amount sufficient to reduce any organ toxicity otherwise occurring and attributable to a).

It will be apparent that the present invention may alternatively be viewed as a method of reducing organ toxicity, e.g. hepatotoxicity or, in particular, nephrotoxicity of a component a) [i.e. for the reduction of organ damage, e.g. liver or, in particular, kidney damage, induced by a component a)] in a subject receiving therapy with a), comprising administration of component b).

Synergistic interaction between immunosuppressant agents, for example Cyclosporine, and compounds of the invention, for example Compound A, in relation to immuno-suppressive activity may be demonstrated in clinical trials and in animal test models, e.g. in the localised graft-versus-host reaction in the rat, performed in accordance with the methods described by Ford et al., Transplantation 10, 258-266 (1970). In this test method, concomittant treatment with an immunosuppressant agent, e.g. a cyclosporin, e.g. Cyclosporine, and compounds of the invention (with treatment with the compound of the invention commencing before inoculation with stimulator cells) results in an inhibition of the graft-versus-host reaction which is greater than the sum of the activities observed when the compounds are administered individually.

Additionally the combination of a) and b) shows a reduction of anti-body titer to sheep red blood cells in the above test I. For example administration of 36 mg/kg/day s.c. of Cyclosporine with 0.1 mg/kg/day of compound A leads to a reduction of 62.6% IgM titre and 79.1 % of the IgG titre. The combination of Cyclosporine (60 mg/kg/day p.o.) and Compound C (0.1 mg/kg/day p.o.) leads to a reduction of 63% of the IgM titre.

Appropriate dosages of other compounds of the invention may be found on the basis of relative potencies of prolactin secretion inhibitor acitivity. Examples of doses are from 0.1 to 10 mg/kg/day s.c.

A further test to show the immunosuppressant activity of the combination is as follows:-

TEST 6: GRAFTING OF NEONATAL HEART TISSUE INTO EARS OF MICE.

In this in-vivo test the rejection time of grafted neonatal heart tissue was increased by the compounds of the invention.

In one method a small incision was made at the base of the ear of a C 57 BL mouse ($H-2^b$). Blunt or fine tweezers were used to loosen the dorsal layer of skin from the cartilaginous layer in order to form a pocket. One half of a heart taken from 1 to 2 day old Balb/c mouse ($H-2^d$) was then inserted in the pocket. At 6 days post grafting and every week thereafter, the heart graft survival was monitored with microelectrodes placed on each side of the graft. A dynograft recorded was used to record electrical activity emanating from the graft. The end point of graft survival was taken as the time when the electrical activity was lost. Untreated mice rejected the implant after 8 1/2 days.

In this model effective immunosuppression was considered to have occurred if the time to rejection was increased to at least 17 days. The time to rejection of the heart allograft was increased by the compounds of

the invention when applied at a dose of from 0.05 to 0.5 mg/kg s.c. on co-administration of cyclosporine (18 mg/kg/day s.c.). With the compound A at 0.05 mg/kg/day, s.c.), 50 per cent of the heart allografts in a group of 10 animals were active on day 17.

In accordance with the method of the present invention, the immunosuppressant agent and compound of the invention may be administered separately and at different times during the course of therapy or subtantially concomitantly. Thus, treatment with the compound of the invention may commence prior to treatment with the immunosuppressant agent, or subsequent to commencement of treatment with the immunosuppressant agent, e.g. after remission of the disease has been effected. The present invention is to be understood as embracing all such regimes of treatment and the term "conjunct administration" is to be interpreted accordingly. In practicing the method of the invention commencement of administration of the compound of the invention prior to treatment with the immunosuppressant agent may, in some instances and depending on e.g. the purpose of therapy with the immunosuppressant agent and the condition of the subject to be treated, be especially advantageous. Thus, commencement of treatment with the compound of the invention up to 7 days or more in advance of treatment with the immunosuppressant agent, may provide an additional and beneficial pre-protective effect against side effects of the immunosuppressant agent, e.g. organ toxicity, e.g. nephrotoxicity.

Alternatively immunosuppressant agents may be administered at the normal dose with the compound of the invention and the cyclosporin dose reduced some weeks, e.g. 1 month after starting therapy with the compound of the invention.

Doses of immunosuppressant agent may be administered in practicing the method of the present invention will of course vary depending upon, e.g. the particular cyclosporin employed, the mode of administration, the condition to be treated (e.g. whether treatment is for the purposes of immunosuppression or otherwise, and if for immunosuppression whether for use in relation to e.g. organ transplant, bone-marrow transplant, or the treatment of auto-immune disease), as well as the effect desired. In addition, dosaging will generally require adjustment for individual patients in order to establish an appropriate long-term drug serum concentration, e.g. by administration of an initial daily starting or "loading"dose with subsequent dose adjustment (generally dose reduction) in accordance with serum levels, e.g. as determined by regular RIA monitoring.

In general, however, where immunosuppressive treatment is required (i.e. where there is potentiation of activity by the conjointly administered compound of the invention satisfactory results are obtained on administration in a dose range of from abut 1 to about 25 mg/kg/day (e.g. in the case of Cyclosporine, from about 5 to about 15 mg/kg/day), administered to the patient orally once or in divided doses 2 or 3 times a day. Where i.v. administration is required, e.g. administration by infusion (for example, in the initial phase of treatment), lower dosages, e.g. of the order of from about 0.5 to about 5.0 mg/kg/day (e.g. in the case of Cyclosporine, from about 1 to about 3 mg/kg/day for an initiating dose, or to about 2 mg/kg/day for a maintainance dose) are generally indicated.

Where treatment is for non-immunosuppressive purposes, satisfactory results are obtained on administration in a dose range of from about 5 to about 50 mg/kg/day (e.g. in the case of Cyclosporine, from about 10 to about 20 mg/kg/day during the initial phase of therapy, reducing to a maintainance dose of from about 5 to about 10 mg/kg/day) administered to the patient orally once or in divided doses 2 or 3 times a day. Where i.v. administration is required, e.g. administration by infusion (for example, in the initial phase of treatment) lower dosages, e.g. of the order of from about 1 to about 10 mg/kg/day (e.g. in the case of Cyclosporine, from about 3 to about 5 mg/kg/day for an initiating dose, or to about 2.5 mg/kg/day for a maintainance dose) are generally indicated.

Doses of the compound of the invention to be administered in practising the method of the present invention will also vary, e.g. depending on the relative prolactin secretion inhibiting potencies of the compound selected, the mode of administration, the cyclosporin therapy applied (e.g. whether immunosuppressive or otherwise/cyclosporin dosaging required) and the particular cyclosporin employed. Administration may be enteral (e.g. oral) or parenteral (e.g. i.m.) or the compound may be administered in sustained release form. Doses for the compounds of the invention e.g. compound C are as given above for sale therapy. For the compound A, suitable daily dosage for oral administration will be of the order of from about 0.1 to about 1 mg/kg/day, administered once or in divided doses 2 or 3 times a day.

Both oral and parenteral dosages forms for cyclosporins, e.g. Cyclosporine, and compounds of the invention, suitable for use in practising the method of the invention, are known in the art e.g. UK Patent Specification GB 2,015,339 B and are commercially available.

Preferred indications include transplantation, Diabetes type I, Uveitis posterior, Rheumatoid Arthritis, and Nephrotic syndrome.

If desired combination dosage forms of the compound of the invention and cyclosporin may be made. These may be based on the drink solution formulation of cyclosporin, i.e.

200 mg Cyclosporine
1 ml Labrafil M 1944 CS (As described in GB 2,015,339) /ethanol mixture (40:15 v/v
0.4 ml Olive oil or Corn oil

which may contain a compound of the invention in free base form, e.g. 0.1 mg compound A or 0.005 mg compound C. The mixture can be used as a Drink Solution or filled into soft gelatine capsules.

## Claims

1. Use of a low molecular weight quinoline for the preparation of an immunosuppressant pharmaceutical composition.

2. Use of a low molecular weight quinoline for the preparation of a pharmaceutical composition suitable for immunosuppressant therapy for co-administration with another immunosuppressant agent.

3. Use of a low molecular weight quinoline for the preparation of a pharmaceutical composition suitable for co-administration with another immunosuppressant agent to lower the side effects caused by the other immunosuppressant agent.

4. Use according to claim 1, 2 or 3 wherein the pharmaceutical composition is for the treatment of one of the following conditions: aplastic anaemia, pure red cell anaemia, idiopathic thrombocytopaenia, systemic lupus erythematodes, polychontritis, sclerodoma, Wegener granulamatosis, dermatomyositis, chronic active hepatitis, myasthenia gravis, psoriasis, Steven-Johnson syndrome, idiopathic sprue, Crohn's disease, Graves opthalmopathy, sarcoidosis, multiple sclerosis, primary billiary cirrhosis, primary juvenile diabetes, uveitis posterior, nephrotic syndrome, interstitial lung fibrosis and psoriatic arthritis.

5. Use according to claim 1, 2 or 3 wherein the pharmaceutical composition is for the treatment of one of the following conditions: arthritis, multiple sclerosis, myasthemia gravis and psoriasis.

6. Use according to any one of the preceeding claims wherein the low molecular weight quinoline is a low molecular weight ergoline derivative.

7. Use according to any one of the preceeding claims wherein the quinoline is 6-n-propyl-8α-(n,N-diethylsulphamoylamino)-ergoline-I.

8. Use according to any one of the preceeding claims wherein the quinoline is N,N-diethyl-N'-[3α,4aα,10aβ)-1,2,3,4,4a,5,10,10a-octahydro-[6-hydroxy-1-propyl-3-benzo[g]quinolinyl] sulfamide.

9. Use according to any one of claims 1 to 4 wherein the quinoline is chosen from the following (5R,8S,10R)-2,6-dimethyl-8α-pivaloylamino-ergoline, (5R,8S,10R)-2-chloro-6-methyl-8α-pivaloylamino-ergoline, lisuride, pergolide, terguride, proterguride, 2-bromoerguride, metergoline, dasergoside, FCE 21336 and GYK1 322887.

10. Use according to any one of the claims 6 to 9 wherein the quinoline is administered in the form of a pharmaceutically acceptable acid addition salt.

11. Use according to any one of claims 2 to 10 wherein the immunosuppressant agent is a cyclosporin.

12. Use according to any one to claims 2 to 10 wherein the immunosuppressant agent is cyclosporine.

13. Use substantially as hereinbefore described with reference to any one of the examples.

14. A pharmaceutical composition containing a low molecular weight quinoline and an immunosuppressant agent.